# EUROPEAN PATENT SPECIFICATION

(11) **EP 1 212 997 B1**
(45) Date of publication and mention of the grant of the patent: **28.03.2007**
(21) Application number: 01128230.8
(22) Date of filing: 28.11.2001
(51) Int. Cl.: A61F 13/475, A61F 13/15

(54) **An absorbent article with leakage safety**
Absorbierender Artikel mit Auslaufsicherheit
Article absorbant avec protection contre les fuites

(30) Priority: 08.12.2000 SE 0004539
(43) Date of publication of application: 12.06.2002
(73) Proprietor: SCA Hygiene Products AB, 405 03 Göteborg (SE)
(72) Inventor: Österdahl, Eje, 42558 Västra Frölunda (SE); Johansson, Asa, 41744 Göteborg (SE); Husmark, Ulrika, 43538 Mölnlycke (SE); Gustafson, Ingrid, 43031 Asa (SE); Stoltze, Anna, 41664 Göteborg (SE)
(74) Representative: Hyltner, Jan-Olof

(56) References cited:
- EP-A- 0 768 072
- WO-A-00/00121
- WO-A-00/19956
- WO-A-00/37002
- WO-A-96/19170
- WO-A-97/40796
- WO-A-99/17813
- WO-A-99/45099
- US-A- 5 662 633

## Description

### FIELD OF INVENTION

The present invention relates to an absorbent article, such as a sanitary napkin, a panty liner or an incontinence protector, which is intended to be carried in the crotch region of the wearer inside the wearer's panties or pants, said article having a generally elongated shape with two long sides, two short sides, two end-portions; a central portion located between the end-portions, a liquid-permeable casing sheet or top sheet which is intended to lie proximal to the wearer's body in use, a liquid-impermeable casing sheet or backing sheet intended to lie distal from the wearer's body in use, and between said sheets a drainage sheet and an element which forms a hump on the side of the article that lies proximal to the wearer's body in use, as seen in a direction from the liquid-permeable casing sheet towards the liquid-impermeable casing sheet.

### BACKGROUND OF THE INVENTION

Conventional absorbent articles of the aforesaid kind are generally flat. Consequently, when the lower abdomen of the female carrier is not flat problems can occur when donning and wearing such articles. In such cases, abutment of the article with the wearer's body is not of the best and if gaps occur between product and the user's body there is a serious risk that body fluid will leak past the long side-edges of the article. Such leakage is particularly undesirable, since it is very liable to soil the wearer's clothes. Rearward leakage is a particular problem in this respect, which normally occurs when the user lies down, for instance at night.

With the intention of solving this problem, it has been proposed to provide the absorbent articles with a pre-formed hump. Absorbent articles that include humps are described in EP-A-0 419 434, among others. The intention of providing absorbent articles with pre-formed humps is to create contact with the genitals of the wearer in use. Discharged body fluids can be caught immediately on leaving the wearer's body and will be absorbed immediately by the article, without running out over its surface and over the long edges of the article.

Another drawback with flat articles is that when the article is used, it is influenced by forces exerted by the wearer's thighs for instance, so as to wrinkle the product and/or cause the long edges of the product to fold over the liquid-permeable surface thereof. A wrinkled surface and/or inwardly folded long edges of the product will significantly reduce the liquid-permeable surface and in many cases to a size that is insufficient to capture all liquid discharged by the wearer at one time, wherewith leakage may occur.

A conventional way of creating a hump is simply to provide a large amount of absorbent material in the absorbent pad within the area where the hump is desired, and form the hump from this excess material. Humps are most often formed from an absorbent material referred to as cellulose fluff pulp, in other words defibred pulp from, e.g., thermomechanical pulp, chemithermomechanical pulp, or chemical sulphite pulp or sulphate pulp. Such a material, however, is not stable when wet, and consequently a hump comprised of such material will collapse and lose its shape when wet. In order to obtain a hump consisting of cellulose fluff pulp and having sufficient height whilst the article is in use, it is necessary to use so much cellulose fluff pulp in the production of the hump as to cause the hump to be felt uncomfortable by the wearer. Another problem that occurs with an article constructed in accordance with the above DESCRIPTION is that control of the liquid dispersion capacity of the article in the z-direction is lost, because the article loses its shape when wetted. It is also known to produce an article which includes a hump that faces towards the wearer, by placing a moulding or shaping element on top of the absorbent core. One drawback in this respect is that the hump results in inertia in liquid transportation down into the product, due to the fact that the shaping element must be filled with liquid before it releases the liquid to the underlying absorption core, said core having a strong liquid suction and absorbing effect and also a liquid retaining absorption effect. A hump can also be provided on the upper side of the article, by providing a planar article with a shaping element that takes a convex shape in relation to the wearer when the sides of the article in the region of the crotch are subjected to greater loads from the wearer's thighs, see for example US 5,662,633. The drawback with this solution is that the shaping element returns to its original planar state immediately the wearer does not subject the sides of the article to pressure, e.g. when she stands with her. legs apart or sits in a "lotus position", and also because it is difficult to produce a shape that corresponds essentially to the body shape of the wearer, solely by flexural deformation.

An absorbent article of the kind described in the introduction is known from EP-Al-0 768 072. However, in this case, the hump-forming element also constitutes the element that shall absorb and store discharged liquid and comprises a compressible, resilient and wetstable material.

WO 96/19710 and WO 00/37002 discloses wicking layers disposed on top of hump-forming elements.

An object of the present invention is to provide an absorbent article of the aforesaid kind that conforms well to the wearer's body, renders the risk of leakage but slight, and which allows a large volume of liquid discharged at one and the same time to be handled with only a slight risk of leakage.

### SUMMARY OF THE INVENTION

These objects are achieved in accordance with the invention with an article according to Claim 1. Such a hump-forming element can be imparted highly effective liquid acquisition properties, meaning that a large volume of liquid delivered at one and the same time can be managed. The longitudinal cut in the absorption layer and the drainage layer also enables the material to be draped more easily around the hump-forming element, therewith enabling a pointed top on the hump to be obtained more readily.

The cut through the drainage layer and the absorption layer has a length of at least 20 mm, although preferably not greater than the length of the hump-forming element. In one beneficial variant, at least one of the end-portions of the longitudinally extending cut is joined to a further cut in the region of the end-portions of the longitudinally extending cut, these other angles defining between themselves and the imaginary extension of the longitudinal cut an angle γ of between 10°-90°, said other cut having a length of between 3-25 mm, preferably between 5-15 mm.

The hump-forming element is preferably comprised of a pressure yieldable, preferably resilient material, that may be an absorbent or a non-absorbent material.

In a further embodiment of the invention, the density of the hump-forming element increases successively away from the absorption layer in a direction towards the liquid-impermeable casing sheet.

The absorption layer will conveniently extend outwardly of at least a part of the contour line of the hump-forming element, but inwardly of the contour line of the drainage sheet. The absorption layer is placed under a drainage layer, because rapid acquisition of liquid into the hump is desired, which contributes to a drier and more comfortable surface proximal to the wearer, and provides a softer, more comfortable hump.

The absorption layer may consist of a dry-formed sheet that contains 5-100% cellulose fibre and which has a density of between 100-300 g/cm³, preferably between 200-250 g/cm³ and a weight by surface area of between 30-2000 g/m². The sheet will have been formed by compressing a web containing cellulose fibre in the absence of subsequent defibration and fluff forming, or by a compressed cellulose foam sheet that has a density of between 0.2-2.0 g/cm³.

The hump preferably has an elongate form and narrows in a direction towards the end-portions of the article and, when seen from one short side of the article, preferably has a triangular cross-section that has a greater width at the base than at the top and preferably a length of between 20 mm and 140 mm and a height between 5-20 mm. The hump may be disposed generally in the centre part of the article or the rear end-portion of the hump may extend into the rear end-portion of the article. The forward end-portion of the hump will preferably not extend into the region of the front end-portion of the article. In the case of such an embodiment, the hump will lie against the wearer's crotch and will continue rearwards between the wearer's buttocks so as to contribute towards an effective seal, primarily when the wearer lies down, for instance to sleep.

### BRIEF DESCRIPTION OF THE DRAWINGS

The invention will now be described in more detail with reference to the accompanying drawings, in which
Figure 1 is a plan view of a sanitary napkin according to a first embodiment of the invention, seen from the side that lies proximal to the wearer in use;
Figure 2 is a longitudinally sectioned view of the sanitary napkin shown in Figure 1, taken on the line II-II;
Figure 3 is a cross-sectional view of the sanitary napkin in Figure 1, taken on the line III-III in said Figure;
Figures 4 and 5 illustrate examples of cuts in the drainage layer and the absorption layer;
Figure 6 is a cross-sectional view similar to Figure 3 and shows another variant of a hump according to the second embodiment of the invention;
Figure 7 is a cross-sectional view similar to that of Figure 3 in which liquid dispersion in the hump is drawn schematically; and
Figure 8 is a cross-sectional view similar to that of Figure 6, where liquid dispersion in the hump is drawn schematically.

### DESCRIPTION OF EXEMPLIFYING EMBODIMENTS

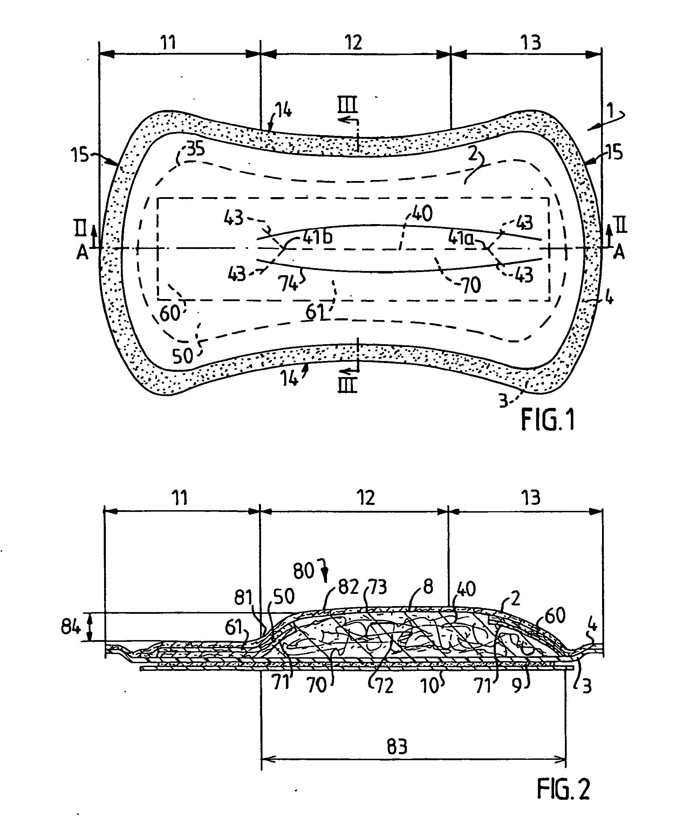

Although absorbent articles in the form of a sanitary napkin are described in the following exemplifying embodiments, it will be understood that these embodiments can also apply to a panty liner or female incontinence napkin. Figure 1 illustrates a sanitary napkin 1 that has a generally elongate shape, with a longitudinal direction, a transversal direction, two long sides 14, two short sides 15, two end-portions 11, 13 and a central portion 12 located between said end-portions.

The sanitary napkin 1 shown in Figures 1, 2 and 3 includes a liquid-permeable casing sheet or top sheet 2 disposed on that side of the napkin 1 which is intended to lie proximal to the wearer in use. The liquid-permeable casing sheet 2 will conveniently consist in a somewhat soft, skin-friendly material. Different types of non-woven material are examples of suitable liquid-permeable materials. Other casing sheet materials that can be used are perforated plastic films, net, knitted, crocheted or woven textiles, and combinations and laminates of the aforesaid types of material.

The sanitary napkin 1 also includes a liquid-impermeable casing sheet or backing sheet 3, disposed on that side of the napkin 1 distal from the wearer in use. The liquid-impermeable casing sheet 3 is conventionally comprised of thin plastic film. Alternatively, there may be used a liquid-permeable material that has been rendered impermeable to liquid in some way or another. For instance, the liquid-permeable material may be coated with a glue that is impermeable to liquid, and the liquid-permeable layer laminated with a liquid-impermeable material, or hot-calendering a material that was initially liquid-permeable, such as to melt down the surface of the material and therewith obtain a liquid-impermeable layer. Alternatively, there may be used other textiles comprised of hydrophobic fibres and so impervious as to enable them to be used as a liquid barrier layer. The liquid-impermeable casing sheet 3 may beneficially be vapour permeable.

The two casing sheets 2, 3 form a joining edge 4 that projects outwardly around the napkin contour line, and are mutually joined at this edge. The sheets may be joined together by means of any appropriate conventional technique, such as gluing, welding or sewing.

A drainage layer 50, absorption layer 60, and a hump-forming element 70 are disposed between the casing sheets 2, 3 as seen in a direction from the liquid-permeable casing sheet 2 towards the liquid-impermeable casing sheet 3.

The hump-forming element 70 forms together with the casing sheet 2, the drainage sheet 50 and the absorption layer 60 a central hump 80 that projects up from the plane of the napkin on that side of the napkin intended to lie proximal to the wearer in use. The central hump 80 is intended to lie against the wearer's body. The central hump 80 may have an elongate shape and may conveniently narrow or taper in a direction towards the end-portions 11, 13 of the napkin. The hump 80 has a generally triangular cross-section. In other words, when seen from one short side of the absorbent article, the hump has a greater width at the base 81 than at the top 82 The hump will preferably have a length 83 of between 20 mm and 140 mm, and a height 84 of between 5-20 mm.

The hump-forming element 70 is comprised at least partially of a pressure yieldable material, preferably a resilient material, although not necessarily an absorbent material. At least that part of the surface 71 of the hump-forming element that lies against the overlying absorption layer 60 has larger capillaries than the absorption layer or sheet 60. Because of the difference in capillary size between the absorbent layer 60 and the surface 71 of the hump-forming element 70 that lies against the absorption layer 60 (the absorption layer 60 has smaller capillaries than the material of the hump-forming element 70), there is no natural dispersion of liquid from the absorption layer 60 to the underlying hump-forming element 70 before the absorption layer reaches saturation.

The drainage layer 50 of the Figure 1 embodiment is wider and longer than the absorption layer 60 and conveniently follows the outer contour of the periphery on the absorbent product inwardly of the joining region 4 between the two casing sheets 2, 3. This creates an absorption acquisition area 61 around the hump base 81 as a safety zone in the event that there is insufficient time for liquid to be absorbed at the top 82 of the hump 80. This non-absorbed liquid would then run down towards the base 81 of the hump 80 and there be absorbed in the safety area 61.

Material suited for the drainage layer 50 is dry-defibred cellulose admixed with an adhesive type binder, or bonding fibres, tissue material, or non-woven material having a capillary size that is in accord with the above reasoning.

The absorption layer 60 placed beneath the drainage layer 50 and draped over the hump-forming element 70 shall constitute the layer capable of receiving and storing essentially all liquid discharged by the wearer. The absorption layer has smaller capillaries than the overlying drainage layer 50. The absorption layer 60 may, for instance, be produced from cellulose pulp. This pulp may exist in rolls, bales or sheets that are dry-defibred and converted in a fluffed state to a pulp mat, sometimes with an admixture of superabsorbents, which are polymers capable of absorbing several times their own weight of water or body liquid (fluid). Examples of other usable materials are different types of foamed materials known, for instance, from SE 9903070-2, natural fibres, such as cotton fibres, peat, or the like. It is, of course, also possible to use absorbent synthetic fibres, or mixtures of natural fibres and synthetic fibres. Patent Application SE 9903070-2 describes a compressed foam material of regenerated cellulose, e.g. viscose. Such foam material will preferably have a density of 0.1 to 2.0 g/cm³.

The absorbent material may also contain other components, such as foam-stabilising means, liquid-dispersing means, or a binder, such as thermoplastic fibres, for instance, which have been heat-treated to hold short fibres and particles together so as to form a coherent unit. A suitable material for the absorbent layer 60 according to the invention is the absorbent material described in WO 94/10956. This material is a dry-formed fibre layer of high density and stiffness that can follow the shape of hump element. In this case, the density will preferably be between 100-300 g/cm³ and preferably between 200-250 g/cm³. This material can be used directly in an absorbent article, without being first defibred. WO 94/10953 describes another, similar material that has particularly suitable properties for blood absorption purposes.

A fastener means 9 in the form of an elongate rectangular region of self-adhesive is provided on the surface of the liquid-impermeable casing sheet 3 that lies distal from the wearer in use. The fastener means 9 extends over the major part of the liquid-impermeable casing sheet 3. The invention is not restricted to the extension of the fastener means 9, and said means may have the form of elongate stripes, transverse regions, dots, circles, or other patterns and configurations. Neither is the invention restricted to the use of solely adhesive fastener means, since friction fasteners may be used and other types of mechanical fasteners, such as press studs, clips, girdles, pants or the like may be used when found suitable to do so.

When a pressure-sensitive adhesive is used to fasten the sanitary napkin in the panties of the wearer, it is usual to cover the adhesive with a removable protective layer 10 which has release properties on at least that side which faces towards the adhesive medium, so as to protect the adhesive against dirt and also to prevent the adhesive from adhering to other, undesired surfaces or against itself until the napkin shall be used.

In the case of the illustrated embodiment6, the hump-forming element 70 extends over large parts of the rear end-portion 13 of the sanitary napkin, which provides a corresponding longitudinally extending hump 80 that projects out from the plane of the napkin on that side thereof intended to lie proximal to the wearer in use. It is particularly beneficial that the end-part 11 placed over the *mons veneris* is free from the hump 80, since the napkin will then conform better to the curvature of the wearer's body. The napkin 1 is intended to be turned towards the wearer so that the hump will lie against the crotch region of the wearer and so that the hump continues rearwardly between the wearer's buttocks and therewith contribute towards an effective seal, primarily when the wearer lies down, for instance to sleep.

Alternatively, the hump 80 may be allowed to extend essentially over a central part of the napkin.

The absorbent layer 60 and the drainage layer 50 have a cut 40 that extends longitudinally along the centre line A-A on the napkin and through which underlying material in the hump-forming element 70 has been pushed through the two material layers, the drainage layer 50 and the absorption layer 60. The length of the cut 40 may be at least 20 mm, preferably not greater than the length of the hump-forming element 70. That part 73 of the hump-forming element that has been pushed up through the cut 40 will beneficially have larger capillaries than the drainage layer. This results in an acquisition surface that presents large liquid-receiving space which enables liquid to quickly enter the napkin, which is to advantage, particularly in those instances when large volumes of liquid are discharged over a short space of time. It is beneficial when that part 73 of the hump-forming element that is pushed up through the absorbent layer 60 and the drainage layer 50 includes a material which will not bind liquid strongly to its fibres but lead the liquid further to the absorption layer 60, so that no liquid will remain close to the liquid-permeable casing sheet 2, such liquid otherwise contributing towards a wet surface against the user's skin. Fibres that can be considered suitable in the part 73 of the hump-forming element 70 are non-absorbent synthetic fibres, absorbent fibres that have been treated to render them hydrophobic and are therewith no longer absorbent.

The provision of a longitudinally extending cut 40 in the drainage layer 50 and the absorption layer 60 enables the triangular profile on the hump 80 to be maintained more easily, even when the hump is subjected to load from the user's body.

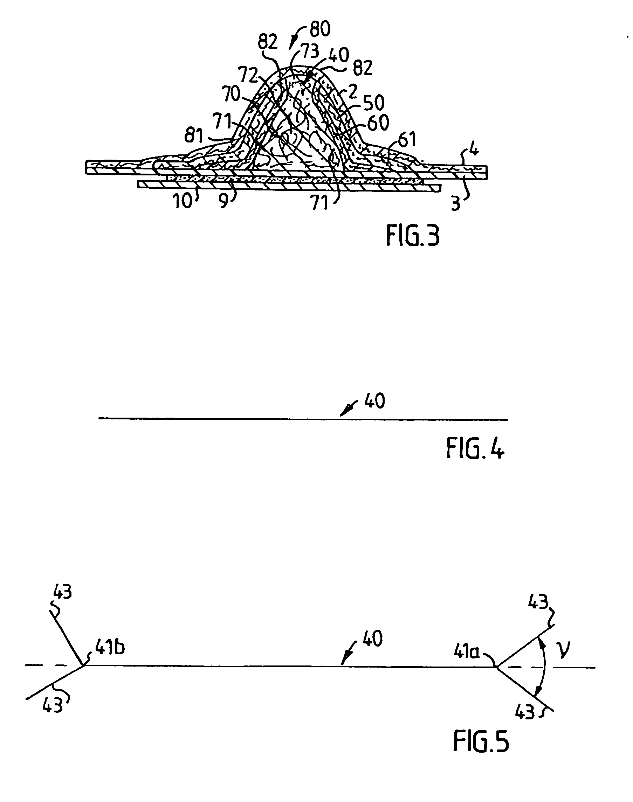

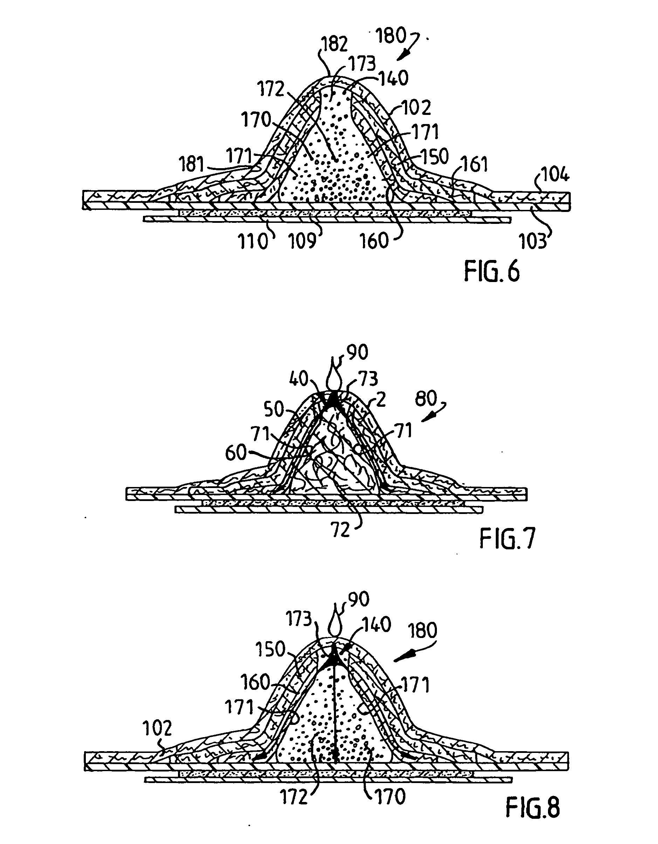

Figures 4 and 5 show examples of cut configurations. The length of the cut 40 is suitably at least 20 mm, although preferably not longer than the length of the hump-forming element 70. The cut 40 may consist solely of a simple longitudinal cut such as that shown in Figure 4, or a cut such as that described in Figure 5. It has been found beneficial to join the end-portions 41a, 41b of the longitudinal cut (see Figure 5) to further cuts 43 that form an angle γ between themselves and the imaginary extension of the longitudinal cut 40, said angle being between 10°-90°. This enables the cut 40 to be opened more easily, so as to enable hump-forming material 70 to be pushed up through both layers, i.e. the drainage layer 50 and the absorption layer 60. The length of the cuts 43 is between 3-25 mm, preferably between 5-15 mm. It is not necessary for the cuts 43 on respective sides on the longitudinal cut 40 to have the same lengths or placements in relation to the longitudinal cut 40, neither is it necessary for the cuts 43 to be made in the same way at the two end-portions 41a, 41b.

In the case of the preferred embodiment, the cut 40 is made without removing material from the drainage layer 50 and the absorption layer 60, which is preferred. It is, of course, feasible to form the cut by providing a cut-out in the layers 50, 60, although this method is not preferred as it complicates manufacture.

Figure 6 is a view similar to the view of Figure 3 and illustrates a second embodiment of a hump 180, where the density of the hump-forming element 170 increases successively down away from the absorbent layer 160 and towards the liquid-impermeable casing sheet 103. In other respects, the components of the Figure 6 embodiment are the same as corresponding components in the embodiment according to Figures 1-3. Consequently, the components of the Figure 6 embodiment have been given the same reference signs as corresponding components in Figure 1-3, to which 100 has been added. The sparsely drawn dots ins Figure 6 are intended to illustrate low density and therewith large capillary sizes, while the tightly packed dots are intended to show successively higher density and therewith successively smaller capillaries. In this way, there is obtained an acquisition area that has an extra large pore structure in the region of the cut 140 up on the top 180 of the hump, wherewith some of the liquid can be drained out and dispersed further by the adjacent absorption layer 160, at the same time as some of the liquid is dispersed and stored within the hump-forming element 170. The description earlier given with reference to Figures 1-3 also applies to the hump 180 in Figure 6, in other words it is beneficial for that part 173 of the hump-forming element that projects up through the absorption layer 160 and the drainage layer 150 to include a material that will not bind the liquid to the fibres of said material too firmly, but that will lead the liquid to the absorption layer 160, so that no liquid that might otherwise contribute to a wet surface against the wearer's skin will remain close to the liquid-permeable casing sheet. It is desirable that at least the central part 172 of the hump-forming element is shape-stable in both a dry and a wet state.

The hump-forming element according to the aforedescribed embodiments may consist of an absorbent structure, cellulose pulp consisting of absorbent or non-absorbent, natural or synthetic fibres, foamed material or material or mixtures thereof. Fibres that can be used to advantage are cellulose fibres of CTMP or CP quality, cotton, rayon, PP fibres, PE fibres and synthetic fibres whose surfaces have been made hydrophilic. The above-described structures, fibres and mixtures thereof may also contain particles of a superabsorbent material that is either distributed uniformly in said material or disposed in layers. By superabsorbent material is meant polymers that exist in the form of fibres, flakes, particles, granules, or the like, and which are capable of absorbing several times their own weight of body liquid whilst swelling and forming a gel. In order to obtain different densities in hump-forming elements 160, the element may be given the form of a mat, i.e. by delivering a flow of relevant fibre material and air to a mould the includes evacuation holes in a number of stages, and thereafter evacuating the air and compressing therebetween parts of the formed hump-forming element 170 to a desired density. Alternatively, layers of mutually different densities may be combined.

Figures 7 and 8 illustrate schematically dispersion of the liquid essentially down into the napkin, the extent of this dispersion being dependent on the variant of hump-forming element chosen for use in the various embodiments.

Figure 7 illustrates a hump that is provided with a cut 40 through the drainage layer 50 and the absoiption layer 60. The surface 71 of the hump-forming element abutting the absorption layer 60 and that part 73 of said element that projects up through the drainage layer 50 and the absorption layer 60 shall at least have a capillary size that is greater than the capillary size of the absorption layer 60. The capillary size of the central part 72 of said element may be smaller than, greater than or the same as the capillary size of the surface 71 of said element that lies against the absorption layer 60. The liquid 90 is absorbed through the casing sheet 2 in z-directions and down into that part of the hump-forming element 3 that has been pushed up through the cut 40 and from there down to the underlying absorption layer 60 where the liquid is dispersed further in said layer and stored therein.

Figure 8 illustrates a hump that is provided with a cut 140 through the drainage layer 150 and the absorption layer 160. The element surface 171 that lies against the absorption layer 160 and that part 173 of said element 170 that projects up through the drainage layer 150 and the absorption layer 160 shall at least have a capillary size that is greater than the capillary size of the absorption layer. The capillary size of the central part 172 of said element decreases gradually in a direction towards the liquid-impermeable casing sheet 103, so as to form a natural absorption gradient down in the central part 172 of the hump-forming element. Liquid 90 is absorbed in z-directions through the casing sheet 102 and down into that part 173 of the hump-forming element 170 that has been pushed up through the cut 140, and from there down to the underlying absorption layer 160 where some of the liquid is dispersed further in said layer 160 and stored therein. As a result of the capillary size gradient, some of the liquid may disperse downwards in the central part 172 of the hump-forming element.

Due to its three-dimensional form adapted to fit the woman body, the article according to the invention is very suitable for carrying an active substance. Examples of suitable active substances are given in WO-A1-99/17813, which is referred to for further details. In WO-A1-99/45099 the use of *Lactobacillus plantarum*, strain LB931, which has been deposited at Deutsche Sammlung von Mikroorganismen, and been assigned accession number DSM11918, in absorbent articles is disclosed. Preferably, LB931 is comprised in the article according to the invention in the area thereof coming in contact with the urogenital region, i.e. perineum, urethra and vagina. The active substance is thus disposed on the area of the article comprising the hump-forming element. The active substance is preferably disposed on the liquid-permeable casing sheet or on the drainage sheet and also on the part of the hump-forming element that projects through the absorbent layer and the drainage layer.

It will be understood that the invention is not restricted to the described and illustrated exemplifying embodiments thereof and that further variants and modifications are conceivable within the scope of the accompanying Claims. All conceivable combinations of the described embodiments are intended to be included by the invention.

### A list of numeric references made in the Figures

1 = Sanitary napkin
2,102 = Liquid-permeable casing sheet
3,103 = Liquid-impermeable casing sheet
4,104 = Joined between the two casing sheets
9,109 = Fastener means
10,110 = Protective layer with release properties for the fastener means
11,111 = The forward end-portion of the napkin
12,112 = The centre portion of the napkin
13,113 = The rear end-portion of the napkin
14,114 = The long sides of the napkin
15,115 = The short sides of the napkin
40,140 = A longitudinally extending cut
41a = One end-part region of the longitudinally extending cut
41b = Another end-part region of the longitudinally extending cut
43 = Cuts at angles to the longitudinally extending cut
50,150 = Drainage layer
51,151 = Drainage layer contour line
60,160 = Absorption layer
61,161 = Absorption receiving range around the base of the hump
70,170 = Hump-forming element
71,171 = Periphery of the hump-forming element
72,172 = Central part of the hump-forming element
73,173 = That part of the hump-forming element that is pushed through the cut
74,173 = The contour line of the hump-forming element
80,180 = A sanitary napkin hump
81,181 = Hump base
82,182 = Top of the hump
85,185 = End-portions of the hump
84,184 = Hump height
83,183 = Hump length
90) Liquid

## Claims

1. An absorbent article in the form of a sanitary napkin, a panty liner or an incontinence protector intended to be carried in the crotch region of the wearer inside the wearer's underclothes, wherein the article has a generally elongate form and includes two long sides (14), two short sides (15), two end-portions (11, 13), a central part (12) between said end-portions, a liquid-permeable casing sheet (2) intended to be turned towards the wearer's body, a liquid-impermeable casing sheet (3) intended to be turned away from the wearer's body, and further including between said sheets (2; 3) in a direction from the liquid-permeable casing sheet (2) towards the liquid-impermeable casing sheet (3), a drainage sheet (50) and a hump-forming element (70) that provides a hump (80) on that side of the article which is proximal to the wearer's body in use also in an uncompressed state of the article, **characterised in that** an absorption layer (60) is disposed between the drainage layer (50) and the hump-forming element (70); and **in that** the absorption layer (60) and the drainage layer (50) include a cut (40) that extends along the longitudinal centre line (A-A) of the article and via which a part of the underlying hump-forming element (70) is projected through the absorption layer (60) and the drainage layer (50), at least that part (71) of the hump-forming element (70) that lies against the overlying absorption layer is comprised of a material that has larger capillaries than the absorption layer; and **in that** the part (73) of the hump-forming element (70) that projects up through the absorption layer (60) and the drainage layer (50) has larger capillaries than the drainage sheet (50), the hump-forming element (70) forms together with the liquid-permeable casing sheet (2), the drainage sheet (50) and the absorption layer (60) said hump (80) that projects up from the plane of the napkin on that side of the napkin intended to lie proximal to the wearer in use.

2. An absorbent article according to Claim 1, **characterised in that** the cut (40) shall have a length of at least 20 mm, but preferably not greater than the length of the hump-forming element (70).

3. An absorbent article according to any one of Claims 1 and 2, **characterised in that** at least one of the end-portions (41a, 341) of the longitudinally extending cut (40) is joined to a further cut (43) in the region of the end-portions (41a, 41b) of said longitudinally extending cut, wherein said further cut defines between itself and the imaginary extension of the longitudinal cut (40) an angle γ of between 10°-90°; and **in that** said further cut (43) has a length of between 3-25 mm, preferably between 5-15 mm.

4. An absorbent article according to any one of Claims 1-3, **characterised in that** the hump-forming element (70) is comprised of a pressure yieldable, preferably resilient, material.

5. An absorbent article according to any one of Claims 1-4, **characterised in that** the hump-forming element (70) is comprised of a non-absorbent material.

6. An absorbent article according to any one of Claims 1-4, **characterised in that** the hump-forming element (70) is comprised of an absorbent material.

7. An absorbent article according to any one of the preceding Claims, **characterised in that** the density of the hump-forming element increases successively in a direction away from the absorption layer and towards the liquid-impermeable casing sheet.

8. An absorbent article according to any one of Claims 1-7, **characterised in that** the absorption layer (60) extends outwardly of at least a part of the contour line (74) of the hump-forming element (70), but inwardly of the contour line (53) of the drainage sheet (5).

9. An absorbent article according to any one of Claims 1-8, **characterised in that** the absorption layer (60) is comprised of a dry-formed sheet containing 5-100% cellulose fibre and having a density of between 100-300 g/cm³, preferably between 200-250 g/cm³ and a weight per unit area of between 30-2000 g/cm², said sheet having been formed by compressing a web containing cellulose fibres in the absence of subsequent defibration and fluff formation.

10. An absorbent article according to any one of Claims 1-8, **characterised in that** the absorption layer (60) is comprised of a compressed sheet of cellulose foam having a density of between 0.2-2.0 g/cm³.

11. An absorbent article according to any one of the preceding Claims, **characterised in that** the hump (80) has an elongate shape and narrows towards the end-portions (11, 13) of said article and, when seen from one short side of the article, said hump preferably presents a triangular cross-section that has a greater width at the base (81) than at the top (82) and preferably a length (83) of between 20 mm and 140 mm and a height (84) between 5-20 mm.

12. An absorbent article according to Claim 11, **characterised in that** the hump (80) is disposed generally in the centre part (12) of the article.

13. An absorbent article according to any one of Claims 1-11, **characterised in that** the rear end-part (85) of the hump extends into the rear end-portion (13) of said article.

14. An absorbent article according to Claim 13, **characterised in that** the forward end-portion (85) of the hump terminates short of the region of the forward end-portion (11) of said article.

15. An absorbent article according to any one of Claims 1-14, **characterised in that** an active substance, such as a lactic acid, is disposed on the liquid-permeable casing sheet or the drainage layer in the area thereof comprising the hump-forming element and on the part of the hump-forming element that projects up through the the absorption layer and the drainage layer.

16. An absorbent article according to Claim 15, **characterised in that** the active substance is *Lactobacillus plantarum,* strain LB931, which has been deposited at Deutsche Sammlung von Mikroorganismen, and been assigned accession number DSM11918.

## Patentansprüche

1. Absorbierender Gegenstand in der Form einer Damenbinde, einer Slipeinlage oder eines Inkontinenzschutzes, der dazu gedacht ist im Schrittbereich des Trägers innerhalb der Unterwäsche des Trägers getragen zu werden, wobei der Gegenstand eine im Wesentlichen längliche Form aufweist und zwei lange Seiten (14), zwei kurze Seiten (15), zwei Endabschnitte (11, 13), einen Mittelteil (12) zwischen den Endabschnitten, eine flüssigkeitdurchlässige Hüllenlage (2), die dazu gedacht ist in Richtung des Körpers des Trägers gewandt zu sein und ein flüssigkeitsundurchlässige Hüllenlage (3), die dazu gedacht ist von dem Körper des Trägers weg gewandt zu sein, umfasst und der ferner zwischen den Hüllen (2, 3) in einer Richtung von der flüssigkeitsdurchlässigen Hüllenlage (2) zur flüssigkeitundurchlässigen Hüllenlage (3), eine Drainagelage (50) und ein einen Höcker bildendes Element (70), das auch in einem unkomprimierten Zustand des Gegenstandes auf der Seite des Gegenstandes einen Höcker (80) bildet, die im Gebrauch proximal des Körpers des Trägers liegt, **dadurch gekennzeichnet, dass** eine Absorptionslage (60) zwischen der Drainagelage (50) und dem den Höcker bildenden Element (70) angeordnet ist; und **dadurch**, dass die Absoprtionslage (60) und die Drainagelage (50) einen Schnitt (40) umfassen, der sich entlang der Längsmittellinie (A-A) des Gegenstandes erstreckt und über den ein Teil des darunter liegenden den Höcker bildenden Elements (70) durch die Absoprtionslage (60) und die Drainagelage (50) vorragt, wobei wenigstens der Teil (71) des dem Höcker bildenden Elements (70), der an der darüber liegenden Absorptionslage anliegt aus einem Material aufgebaut ist, das größere Kapillaren aufweist als die Absorptionslage und **dadurch**, dass der Teil (73) des den Höcker bildenden Elements (70), der durch die Absorptionslage (60) und die Drainagelage (50) vorragt größere Kapillaren aufweist als die Drainagelage (50), wobei das Höcker bildende Element (70) zusammen mit der flüssigkeitsdurchlässigen Hüllenlage (2), der Drainagelage (50) und der Absorptionslage (60) den besagten Höcker (80) bildet, der aus der Ebene der Binde auf der Seite der Binde vorragt, die dazu gedacht ist im Gebrauch proximal des Trägers zu liegen.

2. Absorbierender Gegenstand nach Anspruch 1, **dadurch gekennzeichnet, dass** der Schnitt (40) eine Länge von wenigstens 20 mm, aber nicht länger als das dem Höcker bildende Elements (70), aufweisen sollte.

3. Absorbierender Gegenstand nach einem der Ansprüche 1 und 2, **dadurch gekennzeichnet, dass** wenigstens einer der Endabschnitte (41a, 341) des sich längs erstreckenden Schnittes (40) im Bereich der Endabschnitte (41a, 41b) des sich längs erstreckenden Schnittes mit einem weiteren Schnitt (43) verbunden ist, wobei der weitere Schnitt zwischen sich selbst und dem imaginären Fortsatz des sich längs erstreckenden Schnitts (40) einen Winkel γ zwischen 10° bis 90° definiert und **dadurch**, dass der weitere Schnitt (43) eine Länge zwischen 3-25 mm, vorzugsweise zwischen 5-15 mm aufweist.

4. Absorbierender Gegenstand nach einem der Ansprüche 1-3, **dadurch gekennzeichnet, dass** das den Höcker bildende Element (70) aus einem einen Druck erzeugenden, vorzugsweise elastischen Material aufgebaut ist.

5. Absorbierender Gegenstand nach einem der Ansprüche 1-4, **dadurch gekennzeichnet, dass** das den Höcker bildende Element (70) aus einem nicht absorbierenden Material aufgebaut ist.

6. Absorbierender Gegenstand nach einem der Ansprüche 1-4, **dadurch gekennzeichnet, dass** das den Höcker bildende Element (70) aus einem absorbierenden Material aufgebaut ist.

7. Absorbierender Gegenstand nach einem der vorstehenden Ansprüche, **dadurch gekennzeichnet, dass** die Dichte des den Höcker bildenden Elements in einer Richtung von der Absorptionslage weg und in Richtung der flüssigkeitsundruchlässigen Hüllenlage allmählich zunimmt.

8. Absorbierender Gegenstand nach einem der Ansprüche 1-7, **dadurch gekennzeichnet, dass** sich die Absorptionslage (60) über wenigstens einen Teil der Konturlinie (74) des den Höcker bildenden Elements (70) hinaus aber innerhalb der Konturlinie (53) der Drainagelage (50) erstreckt.

9. Absorbierender Gegenstand nach einem der Ansprüche 1-8, **dadurch gekennzeichnet, dass** die Absorptionslage (60) aus einer trocken formierten Lage aufgebaut ist, die 5-100% Cellulosefasern enthält und eine Dichte zwischen 100-300 g/cm³, vorzugsweise zwischen 200-250 g/cm³ und ein Flächengewicht zwischen 30-2000 g/cm² aufweist, wobei die Lage durch Komprimieren einer Bahn die Cellulosefasern enthält ohne nachfolgende Entfaserung und Fluffbildung formiert wurde.

10. Absorbierender Gegenstand nach einem der Ansprüche 1-8, **dadurch gekennzeichnet, dass** die Absorptionslage (60) aus einer komprimierten Lage aus Celluloseschaum mit einer Dichte zwischen 0,2-2,0 g/cm³ aufgebaut ist.

11. Absorbierender Gegenstand nach einem der vorstehenden Ansprüche, **dadurch gekennzeichnet, dass** der Höcker (80) eine längliche Form hat und sich in Richtung der Endabschnitte (11, 13) des Gegenstandes verjüngt und wobei der Höcker gesehen von einer kurzen Seite des Gegenstandes, vorzugsweise einen dreieckigen Querschnitt aufweist, der eine größere Breite an der Basis (81) als an der Spitze (82) und vorzugsweise eine Länge (83) zwischen 20 mm und 140 mm und eine Höhe (84) zwischen 5-20 mm hat.

12. Absorbierender Gegenstand nach Anspruch 11, **dadurch gekennzeichnet, dass** der Höcker (80) im Wesentlichen im Mittelteil (12) des Gegenstandes angeordnet ist.

13. Absorbierender Gegenstand nach einem der Ansprüche 1-11, **dadurch gekennzeichnet, dass** sich der hintere Endteil (85) des Höckers in den hinteren Endabschnitt (13) des Gegenstandes erstreckt.

14. Absorbierender Gegenstand nach Anspruch 13, **dadurch gekennzeichnet, dass** der vordere Endabschnitt (85) des Höckers kurz vor dem Bereich des vorderen Endabschnitts (11) des Gegenstandes endet.

15. Absorbierender Gegenstand nach einem der Ansprüche 1-14, **dadurch gekennzeichnet, dass** eine aktive Substanz, wie beispielsweise Milchsäure auf der flüssigkeitsdurchlässigen Hüllenlage oder der Drainagelage in dem Bereich davon, der das den Höcker bildende Element umfasst und auf dem Teil des den Höcker bildenden Elements, der durch die Absorptionslage und die Drainagelage vorragt, angeordnet ist.

16. Absorbierender Gegenstand nach Anspruch 15, **dadurch gekennzeichnet, dass** die aktive Substanz ein Lactobacillus plantarum des Stamms LB931 ist, wie er in der deutschen Sammlung von Mikroorganismen hinterlegt wurde und dem die Zugangsnummer DSM11918 zugeteilt wurde.

## Revendications

1. Article absorbant, sous la forme d'une serviette hygiénique, d'un protège slip ou d'une protection contre l'incontinence, prévu pour être porté dans la région de l'entrejambe de l'utilisateur, dans les sous-vêtements de l'utilisateur, **caractérisé en ce que** l'article a généralement une forme allongée et comprend deux côtés longs (14), deux côtés courts (15), deux parties terminales (11, 13), une partie centrale (12) entre lesdites parties terminales, une couche de garniture perméable aux liquides (2) destinée à être tournée vers le corps de l'utilisateur, une couche de garniture imperméable aux liquides (3), destinée à être détournée par rapport au corps de l'utilisateur, et comprenant en outre entre lesdites couches (2, 3) dans une direction à partir de la couche de garniture perméable aux liquides (2) et vers la couche de garniture imperméable aux liquides (3), une couche de drainage (50) et un élément en forme de bosse (70) qui produit une bosse (80) sur le côté de l'article qui est à proximité du corps de l'utilisateur en cours d'usage également dans un état non comprimé de l'art, **caractérisé en ce qu'**une couche absorbante (60) est disposée entre la couche de drainage (50) et l'élément en forme de bosse (70) ; et **en ce que** la couche absorbante (60) et la couche de drainage (50) comportent une incision (40) qui s'étend le long d'une ligne centrale longitudinale (A-A) de l'article et par laquelle une partie de l'élément en forme de bosse sous-jacent (70) fait saillie dans la couche absorbante (60) et la couche de drainage (50), au moins cette partie (71) de l'élément en forme de bosse (70) qui est disposée contre la couche absorbante superposée est composée d'une matière qui a des capillaires plus larges que la couche absorbante ; et **en ce que** la partie (73) de l'élément en forme de bosse (70) qui fait saillie dans la couche absorbante (60) et la couche de drainage (50) a des capillaires plus larges que la couche de drainage (50), l'élément en forme de bosse (70) forme avec la couche de garniture perméable aux liquides (2), la couche de drainage (50), et la couche absorbante (60) ladite bosse (80) qui fait saillie depuis le plan de la serviette sur la face de la serviette prévue pour être disposée à proximité de l'utilisateur pendant l'usage.

2. Article absorbant selon la revendication 1, **caractérisé en ce que** l'incision (40) aura une longueur d'au moins 20 mm, mais de préférence non supérieure à la longueur de l'élément en forme de bosse (70).

3. Article absorbant selon l'une quelconque des revendications 1 et 2, **caractérisé en ce que** au moins une des parties terminales (41a, 341) de l'incision qui s'étend longitudinalement (40) est associée à une incision supplémentaire (43) dans la zone des parties terminales (41a, 41b) de ladite incision qui s'étend longitudinalement, dans lequel ladite incision supplémentaire définit entre elle-même et l'extension imaginaire de l'incision longitudinale (40) un angle γ compris entre 10° et 90° ; et **en ce que** cette dite incision supplémentaire (43) a une longueur comprise entre 3 et 25 mm, de préférence entre 5 - 15 mm.

4. Article absorbant selon l'une quelconque des revendications 1 à 3, **caractérisé en ce que** l'élément en forme de bosse (70) est composé d'une matière, de préférence élastique, déformable par pression.

5. Article absorbant selon l'une quelconque des revendications 1 à 4, **caractérisé en ce que** l'élément en forme de bosse (70) est composé d'une matière non absorbante.

6. Article absorbant selon l'une quelconque des revendications 1 à 4, **caractérisé en ce que** l'élément en forme de bosse (70) est composé d'une matière absorbante.

7. Article absorbant selon l'une quelconque des revendications précédentes, **caractérisé en ce que** la densité de l'élément en forme de bosse augmente successivement dans une direction en s'éloignant de la couche absorbante et vers la couche de garniture imperméable aux liquides.

8. Article absorbant selon l'une quelconque des revendications 1 à 7, **caractérisé en ce que** la couche absorbante (60) s'étend vers l'extérieur d'au moins une partie de la ligne de contour (74) de l'élément en forme de bosse (70), mais vers l'intérieur de la ligne de contour (53) de la couche de drainage (5).

9. Article absorbant selon l'une quelconque des revendications 1 à 8, **caractérisé en ce que** la couche absorbante (60) est composée d'une couche formée à sec qui contient de 5 à 100 % de fibre de cellulose et qui a une densité comprise entre 100 et 300 g/cm³ de préférence entre 200 et 250 g/cm³ et un poids par unité de surface compris entre 30-2000 g/cm², ladite couche ayant été formée en compressant un tissu contenant des fibres de cellulose en l'absence de défibrage intérieur et de formation de flocons.

10. Article absorbant selon l'une quelconque des revendications 1 à 8, **caractérisé en ce que** la couche absorbante (60) est composée d'une couche compressée de mousse de cellulose qui a une densité comprise entre 0,2 et 2,0 g/cm³.

11. Article absorbant selon l'une quelconque des revendications précédentes, **caractérisé en ce que** la bosse (80) a une forme allongée et se réduit vers les parties terminales (11, 13) dudit article et, lorsqu'elle est vue depuis un côté court de l'article, ladite bosse présente de préférence une section transversale triangulaire qui a une largeur plus importante au niveau de la base (81) qu'au sommet (82), et a de préférence une longueur (83) comprise entre 20 mm et 140 mm et une hauteur (84) comprise entre 5 et 20 mm.

12. Article absorbant selon la revendication 11, **caractérisé en ce que** la bosse (80) est placée généralement dans la partie centrale (12) de l'article.

13. Article absorbant selon l'une quelconque des revendications 1 à 11, **caractérisé en ce que** la partie terminale arrière (85) de la bosse s'étend dans la partie terminale arrière (13) dudit article.

14. Article absorbant selon la revendication 13, **caractérisé en ce que** la partie terminale avant (85) de la bosse s'arrête juste avant la zone de la partie terminale avant (11) dudit article.

15. Article absorbant selon l'une quelconque des revendications 1 à 14, **caractérisé en ce qu'**une substance active, telle qu'un acide lactique, est disposée sur la couche de garniture perméable aux liquides ou la couche de drainage dans la zone de celle-ci qui comprend l'élément en forme de bosse et sur la partie de l'élément en forme de bosse qui fait saillie dans la couche absorbante et la couche de drainage.

16. Article absorbant selon la revendication 15, **caractérisé en ce que** la substance active est *Lactobacillus plantarum,* souche LB931, qui a été déposé à la Deutsche Sammlung von Mikroorganismen, et a reçu un numéro d'accès DSM11918.
